# EUROPÄISCHE PATENTSCHRIFT

(11) **EP 3 772 344 B1**
(45) Veröffentlichungstag und Bekanntmachung des Hinweises auf die Patenterteilung: **25.08.2021**
(21) Anmeldenummer: 20187721.4
(22) Anmeldetag: 24.07.2020
(51) Int. Cl.: A61B 17/29, A61B 17/00, A61B 90/50

(54) **MEDIZINISCHES INSTRUMENT**
MEDICAL INSTRUMENT
INSTRUMENT MÉDICAL

(30) Priorität: 05.08.2019 DE 102019121099
(43) Veröffentlichungstag der Anmeldung: 10.02.2021
(73) Patentinhaber: Karl Storz SE & Co. KG, 78532 Tuttlingen (DE)
(72) Erfinder: Grüner, Sven, 78532 Tuttlingen (DE); Merz, Robin, 78532 Tuttlingen (DE)
(74) Vertreter: Hofmeister, Frank

(56) Entgegenhaltungen:
- WO-A1-2014/133180
- WO-A2-03/105563
- US-A1- 2010 041 945
- US-A1- 2010 042 077
- US-A1- 2013 023 925
- US-A1- 2013 218 141

## Beschreibung

Die Erfindung betrifft ein medizinisches Instrument mit einem hohlen Schaft, einer am proximalen Ende des Schaftes angeordneten Betätigungseinheit und einer am distalen Ende des Schaftes angeordneten Instrumentenspitze mit einem Instrument, wobei das Instrument über ein axial verschiebbar im Schaft gelagertes Betätigungselement betätigbar ist, das proximalseitig mit der Betätigungseinheit in Wirkverbindung steht und die Instrumentenspitze über einen Gelenkmechanismus relativ zur Längsachse des Schaftes verschwenkbar ist, wobei der Gelenkmechanismus aus am distalen Ende des Schaftes angeordneten Schwenkgliedern besteht, die über in Längsrichtung des Schaftes verlaufende Lenkdrähte so mit einem proximalseitigen Antrieb verbunden sind, dass eine Bewegung des proximalseitigen Antriebs eine entsprechende relative Bewegung der distalseitigen Schwenkglieder und somit ein Verschwenken der Instrumentenspitze verursacht, sowie mit einem Auslösemechanismus, über den die Lenkdrähte unabhängig von der Betätigung des proximalseitigen Antriebs in einen entspannten, die Beweglichkeit der Instrumentenspitze freigebenden Zustand überführbar sind.

Schwenkglieder mit drei, vier oder mehr außenliegenden Lenkdrähten/Lenkseilen für abwinkelbare medizinische Instrumente sind aus der Praxis für handgeführte und/oder robotische Instrumente bekannt. Für eine feinfühlige Steuerung des distalen Endes eines derartigen medizinischen Instruments haben sich viele dünne Lenkdrähte/Lenkseile gegenüber wenigen dickeren Lenkdrähten/Lenkseilen als vorteilhafter erwiesen, da so unter anderem eine gleichmäßigere Kraftverteilung in alle Abwinklungsrichtungen zu erzielen ist und darüber hinaus erlauben dünnere Lenkdrähte/Lenkseile mehr Platz im Innenraum für elektrische Leitungen und dergleichen.

Ein medizinisches Instrument mit über Lenkdrähten/Lenkseilen angesteuerten Schwenkgliedern ist beispielsweise aus der US 2013/0218141 A1 bekannt.

Ein Nachteil dieser bekannten mit Lenkdrähten/Lenkseilen versehenen Instrumente besteht darin, dass das Reinigen der vielen straff gespannt im Schaft angeordneten Lenkdrähte/Lenkseile problematisch ist. Insbesondere bei der Verwendung von Lenkseilen können sich Verunreinigungen zwischen den einzelnen Adern der Lenkseile festsetzen, die nur schlecht zu entfernen sind.

Darüber hinaus besteht bei den bekannten Instrumenten das Problem, dass es bei einem Ausfall des proximalseitigen Antriebs bei abgewinkelter Instrumentenspitze nicht mehr möglich ist, die Instrumentenspitze zurück in die nicht ausgelenkte Position zu überführen und das Instrument über den Trokar aus dem Operationsgebiet zu entfernen. Im schlimmsten Fall muss der Chirurg den Zugang im Patienten deutlich vergrößern, um den Trokar mitsamt dem darin befindlichen abgewinkelten Instrument zu entfernen.

Weiterhin sind aus der US 2010/0041945 A1 sowie der US 2010/ 0042077 A1 medizinische Instrumente bekannt, bei denen eine distalseitige Werkzeugspitze des Schaftes über Schwenkglieder gegenüber der Längsachse des Schaftes abwinkelbar ist, wobei das Abwinkeln der Schwenkglieder über Lenkdrähte erfolgt, die über einen proximalseitigen Antrieb ansteuerbar sind.

Um die Schwenkglieder und somit auch die Werkzeugspitzen ohne weitere Betätigung des proximalseitigen Antriebs in der jeweiligen Position arretieren zu können, weise diese bekannten medizinischen Instrumente, Arretiermechanismen auf, über die die Schwenkglieder und/oder Lenkdrähte blockierbar sind. Eine Entriegelung der Schwenkglieder für den Fall eines Defekts des proximalseitigen Antriebs existier bei diesen bekannten Instrumenten nicht.

Ein weiteres medizinisches Instrument mit einem über Lenkdrähte verschwenkbaren Schaft ist aus der US 2013/0023925 A1 bekannt. Dieses medizinischen Instrument weist ein axial verschiebbar auf dem Schaft angeordnetes Verriegelungsrohr auf, das zum Arretieren des Verschwenkmechanismus über die Schwenkglieder schiebbar ist. Da es zum Betätigen des Verriegelungsrohres aber erforderlich ist, die Schwenkglieder zunächst über deren proximalseitigen Antrieb in eine gestreckte Stellung zu überführen, lässt sich das Verriegelungsrohr im Falle eines Defekts des proximalseitigen Antriebs nicht mehr aktivieren.

Ein weiteres medizinisches Instrument, das in einer abgewinkelten Stellung arretierbar ist, ist aus der WO 03/105563 A2 bekannt.

Ein gattungsgemäßes medizinisches Instrument ist aus der WO 2014/133180 A1 bekannt. Dieses bekante medizinische Instrument weist einen über einen Motor angetriebenen Auslösemechanismus auf, um bei einem Ausfall des Schwenkantriebs die Lenkdrähte in einen entspannten Zustand überführen zu können. Bei einem Totalausfall der Energiezufuhr ist es aber auch bei dieser technischen Ausgestaltung nicht möglich, die Lenkdrähte in einen entspannten Zustand zu überführen, da in diesem Fall auch der motorische Antrieb des Auslösemechanismus nicht mehr zu betätigen ist.

Davon ausgehend liegt der Erfindung die **Aufgabe** zugrunde, ein medizinisches Instrument der eingangs genannten Art so auszugestalten, dass dies einerseits einfach und wirkungsvoll zu reinigen ist und darüber hinaus ein sicheres Entfernen des medizinischen Instruments auch beim Ausfall des proximalseitigen Antriebs gewährleistet.

Die **Lösung** dieser Aufgabenstellung ist erfindungsgemäß dadurch gekennzeichnet, dass der Auslösemechanismus als rein mechanisch arbeitender Auslösemechanismus ausgebildet ist.

Durch die Verwendung des rein mechanisch arbeitenden Auslösemechanismus ist es jederzeit möglich, die Lenkdrähte unabhängig vom proximalseitigen Antrieb in einen entspannten Zustand zu überführen, der einerseits eine bessere Reinigung der ungespannten Lenkdrähte ermöglicht und darüber hinaus sicherstellt, dass die Instrumentenspitze bei entspannten Lenkdrähten beim Herausziehen des medizinischen Instruments aus dem Trokar selbsttätig in die nicht ausgelenkte Position überführbar ist, wenn der proximalseitige Antrieb ausfallen sollte oder sogar ein totaler Ausfall der Energieversorgung vorleigt.

Gemäß einer praktischen Ausführungsform der Erfindung wird vorgeschlagen, dass mittels des Auslösemechanismus ein Schaftrohr des Schaftes zum Entspannen der Lenkdrähte in Richtung der Längsachse des Schaftes verlagerbar ist. Durch das Verschieben des Schaftrohres nach proximal werden die Lenkdrähte am distalen Ende des Schaftes freigegeben, so dass die Lenkdrähte ohne die führende Wirkung des Schaftrohres schlaff herabhängen, wodurch ein freies Bewegen der Instrumentenspitze unabhängig vom proximalseitigen Antrieb ermöglicht wird. In dieser entspannten Position der Lenkdrähte lassen sich die Lenkdrähte und der Innenraum des hohlen Schaftrohres auch besser und gründlicher reinigen als bei gespannten Lenkdrähten.

Mit einer bevorzugten ersten Ausführungsform der Erfindung wird vorgeschlagen, dass der Auslösemechanismus als mechanischer Spannmechanismus ausgebildet ist. Ein mechanischer arbeitender Spannmechanismus ist konstruktiv einfach und wartungsarm herstellbar und betreibbar.

Weiterhin wird erfindungsgemäß vorgeschlagen, dass über den mechanischen Spannmechanismus gleichzeitig alle Lenkdrähte entspannbar sind. Das gleichzeitige Entspannen aller Lenkdrähte des medizinischen Instruments vereinfacht die Handhabung des Auslösemechanismus und ermöglicht im Falle eines Ausfalls des proximalseitigen Antriebs ein schnelles und unproblematisches Entfernen des medizinischen Instruments über den Trokar auch bei zuvor abgewinkelter Instrumentenspitze.

Gemäß einer ersten Ausführungsform zur Ausbildung des mechanischen Spannmechanismus wird erfindungsgemäß vorgeschlagen, dass der mechanische Spannmechanismus aus zwei gelenkig miteinander verbundenen Hebeln besteht, wobei ein erster Hebel mit seinem freien Ende verschwenkbar am Schaftrohr gelagert ist und ein zweiter Hebel mit seinem freien Ende verschwenkbar an der Betätigungseinheit gelagert ist. Die Ausbildung des mechanischen Spannmechanismus als aus zwei verschwenkbaren Hebeln bestehender Mechanismus stellt eine einfach herzustellende und auch einfach zu betätigende Bauform dar.

Gemäß einer zweiten praktischen Ausführungsform zur Ausbildung des mechanischen Spannmechanismus wird erfindungsgemäß vorgeschlagen, dass der mechanische Spannmechanismus als Spanngewinde ausgebildet ist, wobei das Schaftrohr durch eine Drehung um die Längsachse des Schaftes in Richtung der Längsachse des Schaftes verlagerbar ist. Die Ausbildung als Spanngewinde eine einfach aufgebaute und leicht zu handhabende Variante des mechanischen Spannmechanismus dar.

Schließlich wird mit einer zweiten erfindungsgemäßen Ausführungsform zur Ausbildung des Auslösemechanismus vorgeschlagen, dass der Auslösemechanismus aus einem separaten Antrieb für jeden Lenkdraht besteht.

Weitere Merkmale und Vorteile der Erfindung ergeben sich anhand der zugehörigen Zeichnungen, in denen ein Ausführungsbeispiel eines erfindungsgemäßen medizinischen Instruments nur beispielhaft dargestellt ist, ohne die Erfindung auf dieses Ausführungsbeispiel zu beschränken. In den Zeichnungen zeigt:
- Fig. 1: eine perspektivische Seitenansicht eines medizinischen Instruments gemäß dem Stand der Technik;
- Fig. 2: eine vergrößerte Detailansicht des distalen Schaftendes und des proximalen Schaftendes eines erfindungsgemäßen medizinischen Instruments, die Lenkdrähte und den Auslösemechanismus in der gespannten Position darstellend und
- Fig. 3: eine Ansicht gemäß Fig. 2, jedoch die Lenkdrähte und den Auslösemechanismus in der entspannten Position darstellend.

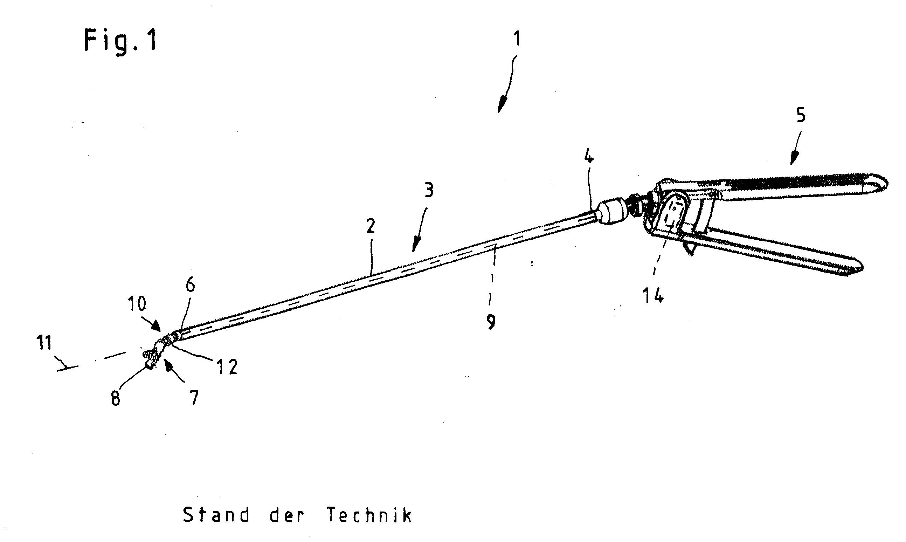

Die Abbildung Fig. 1 zeigt ein medizinisches Instrument 1 mit einem ein hohles Schaftrohr 2 aufweisenden Schaft 3, einer am proximalen Ende 4 des Schaftes 3 angeordneten Betätigungseinheit 5 und einer am distalen Ende 6 des Schaftes 3 angeordneten Instrumentenspitze 7 mit einem Instrument 8, wobei das Instrument 8 über ein axial verschiebbar im Schaftrohr 2 gelagertes Betätigungselement 9 betätigbar ist, das proximalseitig mit der Betätigungseinheit 5 in Wirkverbindung steht.

Bei der Betätigungseinheit 5 kann es sich um eine manuell betätigbare Handhabe oder aber um eine für den robotischen Einsatz ausgelegte, also auch ohne manuelles Zutun betätigbare Baueinheit handeln.

Bei dem Instrument 8 der Instrumentenspitze 7 kann es sich, beispielsweise um ein mit Maulteilen versehenes Werkzeug, wie in Fig. 1 dargestellt, oder aber um ein Endoskop, einen Applikator oder dergleichen handeln.

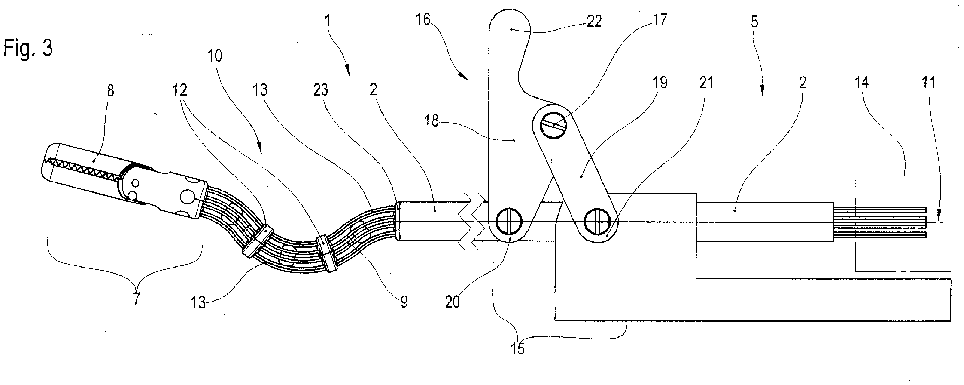

Die Instrumentenspitze 7 ist über einen Gelenkmechanismus 10 relativ zur Längsachse 11 des Schaftes 3 verschwenkbar, wobei der Gelenkmechanismus 10 aus am distalen Ende 6 des Schaftes 3 angeordneten Schwenkgliedern 12 besteht, die über in Längsrichtung des Schaftes 3 verlaufende Lenkdrähte 13 (insbesondere Fig. 3) so mit einem proximalseitigen Antrieb 14 verbunden sind, dass eine Bewegung des proximalseitigen Antriebs 14 eine entsprechende relative Bewegung der distalseitigen Schwenkglieder 12 und somit ein Verschwenken der Instrumentenspitze 7 verursacht.

Auch wenn voranstehend und nachfolgend nur der Begriff Lenkdrähte 13 verwendet wird, können funktional auch Lenkseile verwendet werden, weshalb der verwendete Begriff Lenkdrähte 13 synonym auch als Lenkseil zu lesen und zu verstehen ist.

Ein Nachteil der aus dem Stand der Technik bekannten, mit Lenkdrähten 13 versehenen medizinischen Instrumente 1 besteht darin, dass das Reinigen der vielen straff gespannt im Schaftrohr 2 angeordneten Lenkdrähte 13 problematisch ist. Insbesondere bei der Verwendung von als Lenkseile ausgebildeten Lenkdrähten 14 können sich Verunreinigungen zwischen den einzelnen Adern der Lenkseile festsetzen, die nur schlecht zu entfernen sind.

Darüber hinaus besteht bei den bekannten medizinischen Instrumenten 1 das Problem, dass es bei einem Ausfall des proximalseitigen Antriebs 14 bei abgewinkelter Instrumentenspitze 7 nicht mehr möglich ist, die Instrumentenspitze 7 zurück in die nicht ausgelenkte Position zu überführen und das medizinische Instrument 1 über einen Trokar aus dem Operationsgebiet zu entfernen. Im schlimmsten Fall muss der Chirurg den Zugang im Patienten deutlich vergrößern, um den Trokar mitsamt dem darin befindlichen abgewinkelten medizinischen Instrument 1 zu entfernen.

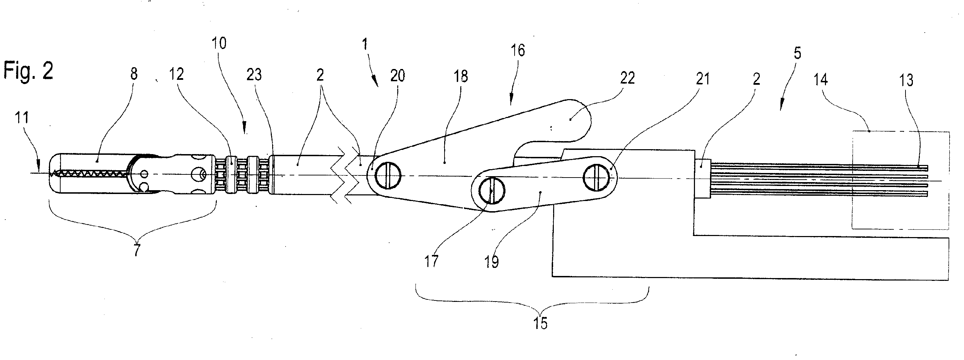

Das in den Abbiidungen Fig. 2 und 3 dargestellte medizinische Instrument 1 weist im Bereich der Betätigungseinheit 5 eine Auslösemechanismus 15 auf, über den den die Lenkdrähte 13 unabhängig von der Betätigung des proximalseitigen Antriebs 14 in einen entspannten, die Beweglichkeit der Instrumentenspitze 7 freigebenden Zustand überführbar sind.

Bei der dargestellten Ausführungsform ist der Auslösemechanismus 15 als mechanischer Spannmechanismus 16 ausgebildet, über den gleichzeitig alle Lenkdrähte 13 des medizinischen Instruments 1 entspannt und wieder gespannt werden können. Dieser mechanische Spannmechanismus 16 besteht aus zwei über eine gemeinsame Schwenkachse 17 gelenkig miteinander verbundenen Hebeln 18 und 19, wobei ein erster Hebel 18 mit seinem freien Ende 20 verschwenkbar am Schaftrohr 2 gelagert ist und der zweite Hebel 19 mit seinem freien Ende 21 verschwenkbar an der Betätigungseinheit 5 gelagert ist. Zum Betätigen des mechanischen Spannmechanismus 16 ist am ersten Hebel 18 ein Griff 22 angeformt.

Die Arbeitsweise des als mechanischer Spannmechanismus 16 ausgebildeten Auslösemechanismus 15 wird nachfolgend anhand der Abbildungen Fig. 2 und 3 erläutert.

Die Abbildung Fig. 2 zeigt das medizinische Instrument 1 in der einsatzbereiten Arbeitsstellung, in der die Lenkdrähte 13 gespannt im Inneren des hohlen Schaftrohres 2 des Schaftes 3 angeordnet sind und die Instrumentenspitze 7 durch Betätigung des in der Betätigungseinheit 5 angeordneten proximalseitigen Antriebs 14 über die Lenkdrähte 13 und die Schwenkglieder 12 gegenüber der Längsachse 11 des Schaftes 3 abgewinkelt werden kann.

In dieser gespannten Position der Lenkdrähte 13 sind die beiden Hebel 18 und 19 des mechanischen Spannmechanismus 16 in Richtung der Längsachse 11 gestreckt hintereinander und im Wesentlichen parallel zum Schaftrohr 2 ausgerichtet.

Das distale Ende 23 des Schaftrohres 2 drückt in dieser Position die frei verschiebbar auf den Lenkdrähten 13 gelagerten Schwenkglieder 12 nach distal hin zur Instrumentenspitze 7. Ein Verschwenken der Instrumentenspitze 7 kann in dieser Position ausschließlich über den proximalseitigen Antrieb 14 bewerkstelligt werden.

Wenn ausgehend von der in Fig. 2 dargestellten gespannten Position der Lenkdrähte 13 der mechanische Spannmechanismus 16 betätigt wird und die beiden Hebel 18 und 19 aufeinander zu verschwenkt werden, wie dies in Fig. 3 dargestellt ist, lässt sich mittels des dargestellten mechanischen Spannmechanismus 16 das hohle Schaftrohr 2 des Schaftes 3 zum Entspannen der Lenkdrähte 13 in Richtung der Längsachse 11 des Schaftes 3 nach proximal verlagern. Die Verlagerung des Schaftrohres 2 nach proximal ist in Fig. 3 am proximalseitigen Überstand des Schaftrohres 2 über die Betätigungseinheit 5 zu erkennen.

Durch das Verschieben des Schaftrohres 2 nach proximal werden die Lenkdrähte 13 am distalen Ende 6 des Schaftes 3 freigegeben, so dass die Lenkdrähte ohne die führende Wirkung des Schaftrohres 2 schlaff aus dem distalen Ende 23 des Schaftrohres 2 herabhängen, wodurch ein freies Bewegen der Instrumentenspitze 7 unabhängig vom proximalseitigen Antrieb 14 ermöglicht wird, wie dies beispielsweise bei einem Ausfall des proximalseitigen Antriebs 14 bei abgewinkelter Instrumentenspitze 7 notwendig sein kann.

In dieser entspannten Position der Lenkdrähte 13 lassen sich die Lenkdrähte 13 und der Innenraum des hohlen Schaftrohres 3 besser und gründlicher reinigen als bei gespannten Lenkdrähten 13.

Die Verlagerung des Schaftrohres 2 des Schaftes 3 in Richtung der Längsachse 11 des Schaftes 3 zum Entspannen der Lenkdrähte 13 kann alternativ zu der dargestellten Ausführungsform als mit den beiden Hebeln 18 und 19 ausgestatteter Spannmechanismus 16 auch durch ein Spanngewinde erzielt werden, wobei das Schaftrohr 2 durch eine Drehung um die Längsachse 11 des Schaftes 3 in Richtung der Längsachse 11 des Schaftes 3 verlagerbar ist.

Weiter alternative Ausführungsformen, um die Verlagerung des Schaftrohres 2 des Schaftes 3 in Richtung der Längsachse 11 des Schaftes 3 zum Entspannen der Lenkdrähte 13 zu erzielen, sind beispielsweise ein Exzenterspanner oder auch ein Rastmechanismus oder dergleichen.

Gemäß einer nicht dargestellten zweiten Ausführungsform zur Ausbildung des Auslösemechanismus 15 kann der Auslösemechanismus 15 aus einem separaten Antrieb für jeden Lenkdraht 13 bestehen.

Ein wie zuvor ausgebildetes medizinisches Instrument 1 zeichnet sich dadurch aus, dass es durch die Verwendung des Auslösemechanismus 15 möglich ist, die Lenkdrähte 13 unabhängig von der Betätigung des proximalseitigen Antriebs 14 zu entspannen.

### Bezugszeichenliste

- 1: medizinisches Instrument
- 2: Schaftrohr
- 3: Schaft
- 4: proximales Ende (Schaft)
- 5: Betätigungseinheit
- 6: distales Ende (Schaft)
- 7: Instrumentenspitze
- 8: Instrument
- 9: Betätigungselement
- 10: Gelenkmechanismus
- 11: Längsachse
- 12: Schwenkglied
- 13: Lenkdraht
- 14: Antrieb
- 15: Auslösemechanismus
- 16: Spannmechanismus
- 17: Schwenkachse
- 18: Hebel (erster)
- 19: Hebel (zweiter)
- 20: freies Ende (erster Hebel)
- 21: freies Ende (zweiter Hebel)
- 22: Griff
- 23: distales Ende (Schaftrohr)

## Patentansprüche

1. Medizinisches Instrument mit einem hohlen Schaft (3), einer am proximalen Ende (4) des Schaftes (3) angeordneten Betätigungseinheit (5) und einer am distalen Ende (6) des Schaftes (3) angeordneten Instrumentenspitze (7) mit einem Instrument (8), wobei das Instrument (8) über ein axial verschiebbar im Schaft (3) gelagertes Betätigungselement (9) betätigbar ist, das proximalseitig mit der Betätigungseinheit (5) in Wirkverbindung steht und die Instrumentenspitze (7) über einen Gelenkmechanismus (10) relativ zur Längsachse (11) des Schaftes (3) verschwenkbar ist, wobei der Gelenkmechanismus (10) aus am distalen Ende (6) des Schaftes (3) angeordneten Schwenkgliedern (12) besteht, die über in Längsrichtung des Schaftes (3) verlaufende Lenkdrähte (13) so mit einem proximalseitigen Antrieb (14) verbunden sind, dass eine Bewegung des proximalseitigen Antriebs (14) eine entsprechende relative Bewegung der distalseitigen Schwenkglieder (12) und somit ein Verschwenken der Instrumentenspitze (7) verursacht, sowie mit einem Auslösemechanismus (15), über den die Lenkdrähte (13) unabhängig von der Betätigung des proximalseitigen Antriebs (14) in einen entspannten, die Beweglichkeit der Instrumentenspitze (7) freigebenden Zustand überführbar sind
**dadurch gekennzeichnet**
**dass** der Auslösemechanismus (15) als rein mechanisch arbeitender Auslösemechanismus ausgebildet ist.

2. Medizinisches Instrument nach Anspruch 1, **dadurch gekennzeichnet, dass** mittels des Auslösemechanismus (15) ein Schaftrohr (2) des Schaftes (3) zum Entspannen der Lenkdrähte (13) in Richtung der Längsachse (11) des Schaftes (3) verlagerbar ist.

3. Medizinisches Instrument nach Anspruch 1 oder 2, **dadurch gekennzeichnet, dass** der Auslösemechanismus (15) als mechanischer Spannmechanismus (16) ausgebildet ist.

4. Medizinisches Instrument nach Anspruch 3, **dadurch gekennzeichnet, dass** über den mechanischen Spannmechanismus (16) gleichzeitig alle Lenkdrähte (13) entspannbar sind.

5. Medizinisches Instrument nach Anspruch 3 oder 4, **dadurch gekennzeichnet, dass** der mechanische Spannmechanismus (16) aus zwei gelenkig miteinander verbundenen Hebeln (18 und 19) besteht, wobei ein erster Hebel (18) mit seinem freien Ende (20) verschwenkbar am Schaftrohr (2) gelagert ist und ein zweiter Hebel (19) mit seinem freien Ende (21) verschwenkbar an der Betätigungseinheit (5) gelagert ist.

6. Medizinisches Instrument nach Anspruch 3 oder 4, **dadurch gekennzeichnet, dass** der mechanische Spannmechanismus (16) als Spanngewinde ausgebildet ist, wobei das Schaftrohr (2) durch eine Drehung um die Längsachse (11) des Schaftes (3) in Richtung der Längsachse (11) des Schaftes (3) nach verlagerbar ist.

7. Medizinisches Instrument nach Anspruch 1, **dadurch gekennzeichnet, dass** der Auslösemechanismus (15) aus einem separaten Antrieb für jeden Lenkdraht (13) besteht.

## Claims

1. Medical instrument with a hollow shaft (3), an actuating unit (5) arranged at the proximal end (4) of the shaft (3), and an instrument tip (7) with an instrument (8) arranged at the distal end (6) of the shaft (3), wherein the instrument (8) is actuatable via an actuating element (9), which is mounted axially displaceably in the shaft (3) and is operatively connected at the proximal end to the actuating unit (5), and the instrument tip (7) is pivotable relative to the longitudinal axis (11) of the shaft (3) via a joint mechanism (10), wherein the joint mechanism (10) is composed of pivoting members (12) which are arranged at the distal end (6) of the shaft (3) and which, via steering wires (13) running in the longitudinal direction of the shaft (3), are connected to a proximal-side drive (14) in such a way that a movement of the proximal-side drive (14) causes a corresponding relative movement of the distal-side pivoting members (12) and thus a pivoting of the instrument tip (7), and also with a trigger mechanism (15) via which the steering wires (13) can be brought, independently of the actuation of the proximal-side drive (14), into a relaxed state that enables the mobility of the instrument tip (7),
**characterized in that**
the trigger mechanism (15) is formed as a purely mechanical trigger mechanism.

2. Medical instrument according to Claim 1, **characterized in that**, by means of the trigger mechanism (15), a shaft tube (2) of the shaft (3) is displaceable in the direction of the longitudinal axis (11) of the shaft (3) in order to relax the steering wires (13).

3. Medical instrument according to Claim 1 or 2, **characterized in that** the trigger mechanism (15) is designed as a mechanical tensioning mechanism (16).

4. Medical instrument according to Claim 3, **characterized in that** all the steering wires (13) can be relaxed simultaneously via the mechanical tensioning mechanism (16).

5. Medical instrument according to Claim 3 or 4, **characterized in that** the mechanical tensioning mechanism (16) is composed of two levers (18 and 19) connected to each other in an articulated manner, wherein a first lever (18) is pivotably mounted with its free end (20) on the shaft tube (2), and a second lever (19) is pivotably mounted with its free end (21) on the actuating unit (5).

6. Medical instrument according to Claim 3 or 4, **characterized in that** the mechanical tensioning mechanism (16) is designed as a tensioning thread, wherein the shaft tube (2), by rotation about the longitudinal axis (11) of the shaft (3), can be displaced in the direction of the longitudinal axis (11) of the shaft (3).

7. Medical instrument according to Claim 1, **characterized in that** the trigger mechanism (15) is composed of a separate drive for each steering wire (13) .

## Revendications

1. Instrument médical comprenant une tige creuse (3), une unité d'actionnement (5) disposée à l'extrémité proximale (4) de la tige (3) et une pointe d'instrument (7) comprenant un instrument (8) et disposée à l'extrémité distale (6) de la tige (3), l'instrument (8) pouvant être actionné par le biais d'un élément d'actionnement (9) qui est monté de manière déplaçable axialement dans la tige (3) et qui est en liaison fonctionnelle du côté proximal avec l'unité d'actionnement (5) et la pointe d'instrument (7) pouvant pivoter par rapport à l'axe longitudinal (11) de la tige (3) par le biais d'un mécanisme d'articulation (10), le mécanisme d'articulation (10) comprenant des éléments pivotants (12) qui sont disposés à l'extrémité distale (6) de la tige (3) et qui sont reliés à un entraînement côté proximal (14) par le biais de fils de direction (13) s'étendant dans la direction longitudinale de la tige (3) de sorte qu'un mouvement de l'entraînement côté proximal (14) génère un mouvement relatif correspondant des éléments de pivotement côté distal (12) et ainsi un pivotement de la pointe d'instrument (7), ainsi qu'un mécanisme de déclenchement (15) permettant de transférer les fils de direction (13) indépendamment de l'actionnement de l'entraînement côté proximal (14) dans un état détendu libérant la mobilité de la pointe d'instrument (7),
**caractérisé en ce que**
le mécanisme de déclenchement (15) est conçu comme un mécanisme de déclenchement purement mécanique.

2. Instrument médical selon la revendication 1, **caractérisé en ce qu'**un tube de tige (2) de la tige (3) pour détendre les fils de direction (13) peut être déplacé dans la direction de l'axe longitudinal (11) de la tige (3) au moyen du mécanisme de déclenchement (15) .

3. Instrument médical selon la revendication 1 ou 2, **caractérisé en ce que** le mécanisme de déclenchement (15) est conçu comme un mécanisme de tension mécanique (16) .

4. Instrument médical selon la revendication 3, **caractérisé en ce que** tous les fils de direction (13) peuvent être détendus simultanément par le biais du mécanisme de tension mécanique (16).

5. Instrument médical selon la revendication 3 ou 4, **caractérisé en ce que** le mécanisme de tension mécanique (16) comprend deux leviers (18 et 19) reliés l'un à l'autre de manière articulée, un premier levier (18) étant monté à son extrémité libre (20) sur le tube d'arbre (2) de manière à pouvoir pivoter et un deuxième levier (19) étant monté à son extrémité libre (21) sur l'unité d'actionnement (5) de manière à pouvoir pivoter.

6. Instrument médical selon la revendication 3 ou 4, **caractérisé en ce que** le mécanisme de tension mécanique (16) est réalisé sous la forme d'un filet tendeur, le tube d'arbre (2) pouvant être déplacé par rotation sur l'axe longitudinal (11) de la tige (3) dans la direction de l'axe longitudinal (11) de la tige (3).

7. Instrument médical selon la revendication 1, **caractérisé en ce que** le mécanisme de déclenchement (15) comprend un entraînement séparé destiné à chaque fil de direction (13).
